# EUROPÄISCHE PATENTANMELDUNG

(11) **EP 4 144 398 A1**
(43) Veröffentlichungstag der Anmeldung: **08.03.2023**
(21) Anmeldenummer: 22166536.7
(22) Anmeldetag: 04.04.2022
(51) Int. Cl.: A61M 37/00, A61H 23/00, A61H 39/08

(54) **HANDGERÄT UND VERFAHREN ZUR HAUTSTRAFFUNG**

(71) Anmelder: Henning, Kathrin, 40479 Düsseldorf (DE)
(72) Erfinder: Henning, Kathrin, 40479 Düsseldorf (DE)
(74) Vertreter: Scheffler, Jörg

(57) **Zusammenfassung**

Die Anmeldung betrifft ein Handgerät (1) und ein Verfahren zur Hautstraffung infolge einer regulierenden Einwirkung auf Hautproteine, indem durch einen antreibbaren Endeffektor (2) zyklisch eine mechanische Belastung auf die Hautoberfläche ausgeübt wird. Die Wirkung beruht nicht auf einer bloßen Andruckkraft auf die Hautoberfläche, sondern auf einem non-invasiven Hautkontakt durch das Eindringen von nadelförmigen, reversierend beweglichen Wirkelementen (5) in einem Hautbereich (3) in die Epidermis. Bei der Anwendung entsteht so über die gesamte, mehrere Wirkelemente (5) einschließende Wirkfläche zyklisch eine homogene Krafteinleitung in die Haut, wobei in jedem Zyklus der Hautbereich (3) in derselben Richtung gedehnt wird.

## Beschreibung

Die Erfindung betrifft ein Handgerät und ein Verfahren zur Hautstraffung mit einem antreibbaren Endeffektor zur Übertragung einer zyklischen mechanischen Belastung auf einen Hautbereich einer Hautoberfläche der menschlichen Haut, um auf ein oder mehrere Hautproteine regulierend einzuwirken.

Allgemein besteht die Haut aus der Epidermis und der Dermis mit einem engen Geflecht von Fasern, die fest mit der Epidermis verflochten sind. Darunter schließt sich die Unterhaut (Tela subcutanea) an.

Bereits ab dem 25. Lebensjahr verlangsamt sich die Zellerneuerung der Haut. Die Epidermis kann weniger Wasser einlagern, da sich die Zusammensetzung im Laufe der Hautalterung verändert, wird dünner und verliert zunehmend an Elastizität.

Falten sind Furchen auf der Oberfläche der Haut, die auf eine Faltung der Epidermis und der darunter liegenden Dermis zurückzuführen sind. Vor allem der Verlust an Feuchtigkeit lässt die Haut schrumpfen und faltig werden. Verantwortlich dafür ist eine altersbedingte Reduzierung von wasserbindenden Bestandteilen, vor allem von Elastin, Kollagen und Hyaluronsäure.

Bei Elastin und Kollagen handelt es sich um sog. Strukturproteine, die von besonderen Zellen, den Fibroblasten, in der Dermis gebildet werden. Elastin und Kollagen haben eine formgebende und -erhaltende Aufgabe. Sie sind von faserartiger Struktur und besitzen eine ausgeprägte Fähigkeit, Wasser zu binden.

Es ist bekannt, dass die einwirkende mechanische Belastung das Zellverhalten beeinflussen kann. Es wurde nachgewiesen, dass mit einem Gerät, das ein oszillierendes Drehmoment und zyklische Belastungen bei verschiedenen Frequenzen erzeugt, höhere Expressionsraten - insbesondere für Decorin, Fibrillin, Tropoelastin und Prokollagen - erreicht werden konnten. Der mechanische Reiz, abhängig von der Stimulusfrequenz, löste eine Anti-Falten-Wirkung aus.

Die EP 3 244 867 B1 betrifft ein Gerät zum Stimulieren eines Hautabschnitts bei einer Stimulationsfrequenz mit einem motorisch angetriebenen Endeffektor, der eine Vielzahl von Kontaktpunkten mit dem Hautabschnitt bildet. Der Endeffektor wird mit einer Oszillationsfrequenz angetrieben, die auf einer Zielstimulationsfrequenz basiert. Durch eine äußere Kraft wird der Endeffektor in Richtung auf den Hautabschnitt vorgespannt und erzeugt so einen zyklischen Reiz in dem Hautabschnitt

Bei der EP 3 244 864 B1 dient ein Gerät der zyklischen mechanischen Belastung innerhalb eines Hautabschnitts dazu, auf ein oder mehrere Hautproteine regulierend einzuwirken, insbesondere mit einer Zyklus- oder Schwingungsfrequenz im Bereich bis 100 Hertz und einer Winkelschwingungsbewegung mit einer Amplitude von etwa 3 Grad bis etwa 17 Grad. Ein Endeffektor hat mehrere Kontaktpunkte und erzeugt Schwingungen, Vibrationen, Hin- und Herbewegungen, Drehungen oder zyklische Bewegungen.

Die EP 3 082 576 B1 betrifft ein kosmetisches Verfahren zur Behandlung der menschlichen Haut durch Ansaugen eines Hautbereichs in eine Kammer, um die Haut mit einer Oberfläche in Kontakt zu bringen und aus einer wiederholten Bewegung der Haut gegen die Oberfläche infolge wiederholter Vakuumzyklen einen Schleifeffekt zu erreichen.

Ferner ist eine mechanische Straffung der Haut mittels LPG-Geräten (LPG-Endermologie) durch mechanische Stimulation bekannt. Das LPG-Gerät hat ein Handstück, das eine subdermale Massage durchführt, wobei durch diese Behandlung die Fibroblasten stimuliert werden, um die Synthese von Kollagen und Elastin zu steigern, was die Festigkeit der Haut verstärkt.

Auch durch motorbetriebene Massagegeräte kann eine Anzahl von nützlichen Wirkungen erzielt werden, einschließlich verbesserter Hautstraffung.

Aus der DE 299 11 555 U1 ist ein Massagegerät bekannt, dass aus zwei im Abstand vertikal übereinander angeordneten, drehend angetriebenen Wellen besteht, an denen drehfest eine Anzahl von Massage- und Knetrollen angeordnet ist.

Aus der DE 20 2015 104 519 U1 ist ein motorbetriebenes Massagegerät bekannt, welches einen Massagekopf zur simultanen Drehung um eine Drehachse und zur Vibration in eine Richtung längs der Drehachse umfasst.

Das Massagegerät gemäß der EP 2 505 177 A1 dient zur Druck- und Streichmassage durch kreisförmige oder elliptische Verschiebetechniken, welches mit leichtem Druck angewandt wird, sich überwiegend auf den Haut- und Unterhautbereich auswirkt und eine Straffung und Festigung von Hautzonen bewirkt.

Die DE 10 2008 046 428 A1 bezieht sich auf ein Hilfsmittel der Kompressionstherapie und beruht auf einer punktuellen Verteilung des Drucks durch eine Aufnoppung des Gewebes, wobei es bei einer optimalen Einstellung der Druckparameter zu einer Rotation der einzelnen Noppen um den fiktiven Auflagepunkt auf der Hautoberfläche kommt.

Beim Microneedling wird mittels beweglicher Mikronadeln, die exakt vertikal über der Haut positioniert werden, die Epidermis mit bis zu 1.900 Stichen pro Sekunde stimuliert. In der Folge setzt die Haut einen Regenerationsmechanismus in Gang, bei dem die Sauerstoffproduktion angeregt und körpereigene Wirkstoffe wie Elastin, Kollagen und Hyaluronsäure ausgeschüttet werden und eine Hautstraffung erreicht werden soll.

Ferner sind Nadelköpfe zum Tätowieren, Einbringen von Permanent Make-up oder Microneedling bekannt, deren Nadel aus einer oder mehreren Einzelnadeln, sogenannten Nadelpaketen, besteht, die mittels eines elektrischen Antriebs entlang ihrer Längsachse beweglich sind. Die Nadeln durchstechen die Epidermis und dringen in die Dermis oder in noch tiefere Hautschichten ein.

Die DE 10 2020 132 614 A1 betrifft einen Nadelkopf zur Behandlung der Epidermis, durch Einbringen einer Flüssigkeit in die Epidermis, mit einer Anlagefläche zur Anlage an die Epidermis und einer axial beweglichen Nadel, die in einem Winkel von weniger als 30 Grad auf die Epidermis trifft. Dadurch wird die Haut nicht aufgestaut und eine besonders gleichmäßige Eindringtiefe wird erzielt, wobei ein tieferes Eindringen in die Haut möglich ist. Zugleich soll das Aufstauen durch die Geometrie der Anlagefläche minimiert sein.

Außerdem ist auch aus der DE 196 17 920 C2 eine Einrichtung zur Behandlung der Haut bekannt, die ein motorisch angetriebenes Nadelband mit einer Vielzahl von punktuell wirkenden und flächig verteilten Nadeln mit stumpfer, gerundeter Spitze aufweist, wobei eine lineare Bewegung parallel zur Auflagestelle durchgeführt wird.

Als nachteilig bei den aus dem Stand der Technik bekannten Endeffektoren erweist sich die mit der Winkelauslenkung des oszillierenden Effektorkopfs ungleichmäßig auf die Haut übertragene Krafteinwirkung, abhängig von dem Abstand bzw. Radius des jeweiligen Kontaktpunkts gegenüber der Drehachse des Effektorkopfs. Dabei sind lokale Überschreitungen der maximalen Wirkkraft nicht zuverlässig auszuschließen. Zudem führt die zyklische Umkehrung der Kraftwirkrichtung bei der Kraftübertragung auf die Haut nach neuen Erkenntnissen nicht zu optimalen Ergebnissen.

Der Erfindung liegt die Aufgabe zugrunde, die Einleitung bzw. Übertragung der zyklischen Belastung weiter zu optimieren und ein verbessertes Zellverhalten zu erreichen. Diese Aufgabe wird erfindungsgemäß mit einem Handgerät und einem Verfahren gemäß den Merkmalen der unabhängigen Ansprüche gelöst. Die weitere Ausgestaltung der Erfindung ist den Unteransprüchen zu entnehmen.

Erfindungsgemäß hat also das Handgerät einen Endeffektor mit einer Vielzahl von reversierend zwischen einer Arbeitsposition und einer Ruhe- bzw. Nichteingriffsposition translatorisch antreibbaren strangförmigen Wirkelementen, die zyklisch in Kontakt mit der Haut treten, sodass zeitweise in der Ruhe- bzw. Nichteingriffsposition der Kontakt zwischen den Wirkelementen und der Haut unterbrochen ist und die Wirkelemente in eine gegenüber der Hautoberfläche beabstandete Position zurückkehren, während in der Arbeitsposition die Wirkelemente non-invasiv auf die Haut einwirken und in die Epidermis eindringen, wobei der Endeffektor und/oder das Handgerät eine zumindest abschnittsweise ebene Anlagefläche zur Anlage mit einer vorbestimmten Orientierung des Endeffektors gegenüber dem Hautbereich aufweist, und die jeweilige Bewegungsachse der translatorischen Bewegung der Wirkelemente einen spitzen Winkel mit der Anlagefläche einschließt, sodass im Gebrauch über die gesamte, mehrere Wirkelemente einschließende Wirkfläche zyklisch eine homogene Krafteinwirkung erzeugt wird, wobei in jedem Zyklus der Hautbereich in Vorschubrichtung vor der Wirkfläche vorübergehend gegenüber der umgebenden Hautfläche gestaucht oder komprimiert wird, indem die Wirkelemente so ausgeführt sind, dass diese in einer ersten Arbeitsphase ihrer translatorischen Bewegung nicht oder nur geringfügig, insbesondere die Epidermis nicht durchdringend in die Haut eindringen und dabei eine maximale Eindringtiefe erreichen, und in einer zweiten Arbeitsphase der fortgesetzten translatorischen Bewegung die Eindringtiefe nicht oder nur geringfügig erhöht ist und dadurch die Haut relativ zu der Anlagefläche in Vorschubrichtung gedehnt wird.

Erfindungsgemäß wird so erstmals eine zyklische mechanische Belastung durch die kombinierte Dehnung und Stauchung bzw. Komprimierung von Hautbereichen erreicht, die jeweils über die gesamte behandelte Fläche homogen mit gleichbleibender Wirkrichtung erfolgt und insbesondere eine maximale Belastung nicht überschreitet. Der wesentliche Erfindungsgedanke beruht dabei auf der Erkenntnis, die Wirkelemente zunächst in die Haut eindringen zu lassen, wobei die Epidermis nicht durchdrungen wird, sodass die Behandlung non-invasiv erfolgt. Dadurch haben die Wirkelemente bereits die maximale Eindringtiefe erreicht mit der Folge, dass eine fortgesetzte translatorische Verlagerung der Wirkelemente oder eine manuell übertragene Vorschubkraft nicht etwa die Eindringtiefe vergrößert, sondern vielmehr die Haut im Bereich der Anlagefläche gedehnt, hingegen der in Vorschubrichtung davorliegende Hautbereich gestaucht wird. Indem die Wirkelemente in die Haut eindringen, beruht dieser Effekt nicht oder zumindest nicht allein auf einem Kraftschluss zwischen dem Endeffektor und der Haut, sondern zu einem wesentlichen Teil auch auf einem Formschluss der in der Haut kurzzeitig verankerten Wirkelemente, wodurch die Dehnung und Komprimierung anders als bei den aus dem Stand der Technik bekannten Behandlungsmethoden um einen vorbestimmten Betrag erfolgt. Damit einher geht auch der wesentlich verbesserte Eintrag der vorzugsweise ergänzend verwendeten Wirkstoffe, die zuvor beispielsweise als Serum auf die Haut auftragen werden und einerseits durch den Nadeleffekt der eindringenden Wirkelemente in die Epidermis gelangen, andererseits unter dem Einfluss der Dehnung und Komprimierung weitaus schneller und effektivier migrieren können.

Zum Verständnis der Erfindung ist zu berücksichtigen, dass die translatorisch reversierende Bewegung der Wirkelemente zwischen der Arbeitsposition und der Ruhe- bzw. Nichteingriffsposition mit einer Frequenz erfolgt, die für den Betrachter optisch kaum wahrnehmbar ist, und der Betrag der translatorischen Bewegung der Wirkelemente wenige Millimeter, vorzugsweise 1 mm oder wenige 1/10 mm, nicht überschreitet, was dazu führt, dass die Krafteinleitung sehr kurzzeitig erfolgt.

Obwohl das Handgerät auf der Haut vorzugsweise in kleinen und/oder großen kreisenden Bewegungen geführt wird, um so die gewünschten Hautbereiche mehrfach zu erreichen, verändert sich die Wirkrichtung der Dehnung oder Komprimierung aufgrund der geringen Amplitude der Bewegung in aufeinanderfolgenden Zyklen nicht oder nur äußerst geringfügig.

Anders als beim Stand der Technik, bei dem der Endeffektor bei jedem Zyklus die Haut sowohl in einer ersten Wirkrichtung als auch in einer genau entgegengesetzten zweiten Wirkrichtung belastet wird, erfolgt daher erfindungsgemäß die Belastung der Haut in den folgenden Zyklen stets mit einer nahezu unveränderten Wirkrichtung. Insbesondere wird also mittels der Wirkelemente eine Kraft in distaler Richtung eingeleitet, hingegen ist eine Krafteinleitung in entgegengesetzter, proximaler Richtung bereits prinzipiell ausgeschlossen, weil die Wirkelemente in proximaler Richtung außer Eingriff mit der Haut kommen und dabei keine Zugkraft auf die Haut übertragen.

Erfindungsgemäß werden nicht nur Hautfalten und Narben geglättet, sondern auch weiches Gewebe, wie beispielsweise Tränensäcke und Schlupflider, gestrafft. Die Haut des Oberlids zieht sich ebenfalls zusammen und somit werden Schlupflider gestrafft. Narben im Augenbereich lassen sich ebenfalls auf diese Weise glätten und werden dadurch wesentlich unauffälliger. Außerdem kann grobporige Haut verfeinert werden.

Die Bewegung der Wirkelemente kann durch die Vorschubkraft infolge der manuellen Betätigung des Handgeräts optimiert werden. Insbesondere kann so die Einwirkungsdauer auf einen bestimmten Hautbereich gesteuert werden, ohne die Eindringtiefe der Wirkelemente zu verändern. Dabei dient die Vorschubkraft vor allem dazu, die Haut zu glätten und eine flächige Auflage der Anlagefläche des Endeffektors zu erreichen, um ein gleichmäßiges Behandlungsergebnis zu erreichen. Die gewünschte hautstraffende Wirkung entsteht hingegen grundsätzlich aufgrund der eindringenden Wirkelemente und deren translatorischer Auslenkung.

Dementsprechend ist es gemäß einer weiteren bevorzugten Variante von Vorteil, wenn die betreffenden Hautbereiche in zueinander orthogonalen Richtungen, beispielsweise sowohl quer als auch längs zu der Orientierung von Hautfalten, mit dieser manuellen Technik behandelt werden.

Selbstverständlich kann die reversierende translatorische Bewegung der Wirkelemente durch manuell eingebrachte, schiebende lange oder kurze Hübe in Vorschubrichtung wirksam unterstützt werden, um so die vorteilhafte Wirkung, insbesondere durch eine zusätzliche Hautdehnung, zu unterstützen. Die maximale Eindringtiefe verändert sich dabei nicht.

Die Wirkelemente könnten abweichend von einer im Wesentlichen zylindrischen Form eine Querschnittserweiterung aufweisen, die als Anlagefläche und Kontaktfläche die Eindringtiefe begrenzt. Besonders vorteilhaft ist es hingegen, wenn die maximale Eindringtiefe durch die maximale vorspringende Arbeitsposition der Wirkelemente gegenüber der Anlagefläche bestimmt ist. Indem in der Arbeitsposition die Anlagefläche auf der Haut aufliegt, kann auch bei unsachgemäßer Handhabung des Handgeräts die Eindringtiefe nicht weiter erhöht werden. Erfindungsgemäß wird so eine problemlose Handhabung des Handgeräts erreicht.

Dabei hat es sich als besonders vorteilhaft erwiesen, wenn die maximale Eindringtiefe einstellbar ist, um so eine optimale Anpassung der Behandlung an verschiedene Hautbereiche und Hauttypen vornehmen zu können. Insbesondere kann die Eindringtiefe innerhalb besonders empfindlicher Hautbereiche oder infolge erkennbarer Hautreaktionen reduziert werden.

Um das verletzungsfreie Eindringen der Wirkelemente in die Haut zu begünstigen, ist es besonders vorteilhaft, wenn die Wirkelemente nadel- oder spindelförmig ausgeführt sind, wobei die Umfangsfläche abschnittsweise glatt und/oder abschnittsweise eine Strukturierung aufweisen kann. Beispielsweise können die Wirkelemente mit einer in Längsrichtung und/oder in Umfangsrichtung verlaufenden Einschnürung oder Aufweitung ausgestattet sein.

Bei einer anderen, ebenfalls besonders praxisgerechten Abwandlung der Erfindung ist die Anlagefläche zur Glättung der Haut infolge der Vorschubkraft quer zu der Vorschubrichtung ausgeführt, indem beispielsweise eine Konturierung der Anlagefläche die Haut im Bereich der Wirkelemente eine Spreizwirkung auf die Haut überträgt, um so die Haut zu glätten. Dadurch kann auch bei Hautunebenheiten eine gleichmäßige Eindringtiefe mehrerer Wirkelemente sichergestellt werden.

Eine andere, ebenso zweckmäßige Ausgestaltungsform der Erfindung wird auch dann erreicht, wenn das Handgerät mit einer Einrichtung zur Begrenzung der manuell übertragenen Vorschubkraft ausgestattet ist, sodass unbeabsichtigte oder unkoordinierte Bewegungen nicht zu einer unerwünschten Einwirkung auf die Haut führen. Die Wirkelemente werden dabei außer Eingriff mit der Haut gebracht.

Vorzugsweise kann die Anlagefläche mit einem Wirkstoffreservoir verbunden sein, sodass eine stets ausreichende Zuführung von Serum zur Hautberuhigung oder als Träger von Wirkstoffen sichergestellt ist.

Weiter kann es auch zweckmäßig sein, wenn die Wirkelemente und/oder die Anlagefläche temperierbar, insbesondere beheizbar, ausgeführt sind, um so eine gleichbleibende Temperatur entsprechend der Körpertemperatur zu ermöglichen, welche die Hautstraffung zusätzlich begünstigt.

Die zweitgenannte Aufgabe wird erfindungsgemäß mit einem Verfahren gemäß den Merkmalen des Anspruchs 2 dadurch gelöst, dass zur Hautstraffung nadel- oder spindelförmige Wirkelemente reversierend translatorisch bewegt werden. In der Ruhe- bzw. Nichteingriffsposition sind die Wirkelemente außer Kontakt mit der Haut und treten in eine gegenüber der Hautoberfläche beabstandete Position zurück.
In der Arbeitsposition wird ein non-invasiver Kontakt der Wirkelemente mit der Haut erzeugt, indem die Wirkelemente in einem spitzen Winkel zu der Hautoberfläche bewegt werden und über die gesamte, mehrere Wirkelemente einschließende Wirkfläche zyklisch eine homogene Krafteinwirkung erzeugen.

Dadurch wird in jedem Zyklus der Hautbereich in Vorschubrichtung vor der Wirkfläche vorübergehend gegenüber der umgebenden Hautfläche gestaucht oder komprimiert und in dem sich anschließenden Hauptbereich entgegen der Vorschubrichtung gedehnt. Diese Hautdehnung beruht auf dem erfindungsgemäßen Effekt, bei dem die Wirkelemente in einer ersten Arbeitsphase ihrer translatorischen Bewegung in die Epidermis eindringen, bis eine maximale Eindringtiefe in der Haut erreicht ist. In der zweiten Arbeitsphase der stetigen translatorischen Bewegung wird die Eindringtiefe nicht oder nur sehr geringfügig erhöht mit der Folge, dass dadurch der Hautbereich relativ zu der Anlagefläche in Vorschubrichtung gedehnt wird.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine Seitenansicht eines erfindungsgemäßen Handgeräts mit mehreren Wirkelementen in einer Ruheposition;
- Fig. 2: eine Seitenansicht des Handgeräts in einer Arbeitsposition der Wirkelemente;
- Fig. 3: eine Draufsicht auf das Handgerät in der in der Figur 2 gezeigten Arbeitsposition.

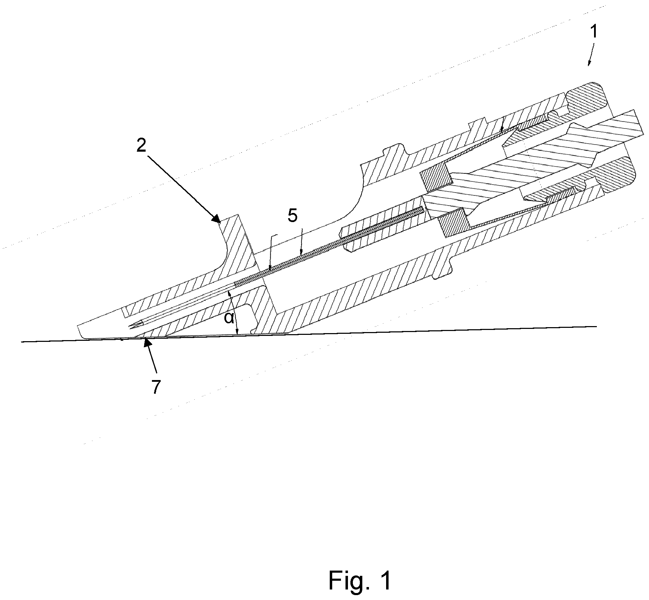

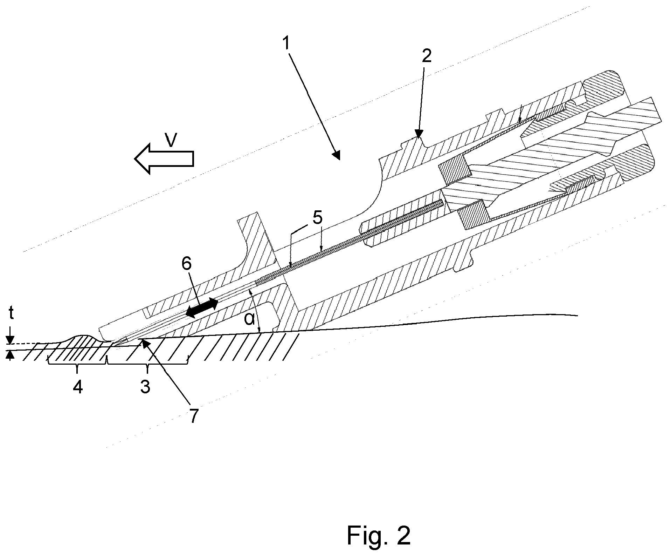

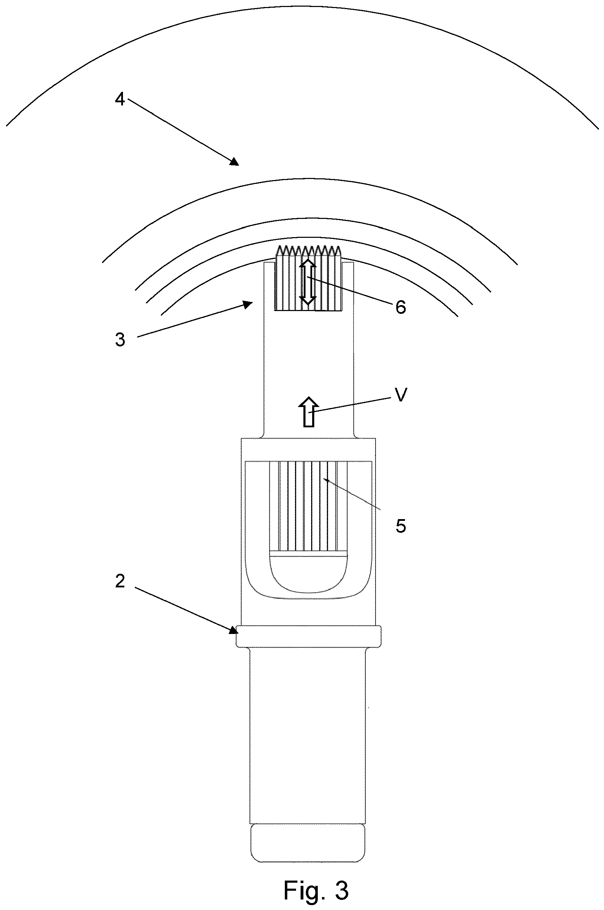

Das erfindungsgemäße Handgerät 1 zur Hautstraffung wird nachstehend anhand der Figuren 1 bis 3 näher erläutert. Das nicht weiter dargestellte Handgerät 1 hat einen Endeffektor 2 zur Übertragung einer zyklischen mechanischen Belastung auf einen Hautbereich 3, 4 einer Hautoberfläche der menschlichen Haut, die ausreicht, um auf ein oder mehrere Hautproteine regulierend einzuwirken. Dabei beruht die Wirkung des Endeffektors 2 nicht auf einer bloßen Andruckkraft auf die Hautoberfläche. Vielmehr sind mehrere Wirkelemente 5 reversierend zwischen einer in der Figur 1 gezeigten Ruhe- bzw. Nichteingriffsposition und einer in den Figuren 2 und 3 gezeigten Arbeitsposition in Pfeilrichtung 6 translatorisch beweglich. Dadurch kommen die Wirkelemente 5 zyklisch in einen non-invasiven Hautkontakt, d. h. die Wirkelemente 5 dringen lediglich bis zu einer maximalen Eindringtiefe tₘₐₓ gegenüber einer Anlagefläche 7 des Endeffektors 2 mit einem spitzen Winkel a in die dargestellte Epidermis ein.

Im Gebrauch entsteht so über die gesamte, mehrere Wirkelemente 5 einschließende Wirkfläche zyklisch eine homogene Krafteinleitung in die Haut, wobei in jedem Zyklus der Hautbereich 3 in Vorschubrichtung V vor der Anlagefläche 7 vorübergehend gegenüber der umgebenden Hautfläche in dem ersten Hautbereich 3 gedehnt und in dem in Vorschubrichtung V davor liegenden Hautbereich 4 gestaucht oder komprimiert wird.

Die so erreichte Dehnung des einen Hautbereichs 3 und die Stauchung bzw. Komprimierung des anderen Hautbereichs 4 erfolgt anders als beim bisher bekannten Stand der Technik über die gesamte behandelte Fläche homogen und in derselben Richtung. Durch das noninvasive Eindringen der Wirkelemente 5 hängt die hautstraffende Wirkung zudem nicht von der Andruckkraft der Anlagefläche 7 gegenüber der Haut ab, was sich vor allem in sehr empfindlichen Hautbereichen 3, 4 bereits als sehr vorteilhaft erwiesen hat.

### BEZUGSZEICHENLISTE

- 1: Handgerät
- 2: Endeffektor
- 3: Hautbereich
- 4: Hautbereich
- 5: Wirkelement

- 6: Pfeilrichtung
- 7: Anlagefläche

- t: Eindringtiefe
- α: Winkel
- V: Vorschubrichtung

## Patentansprüche

1. Handgerät (1) zur Hautstraffung mit einem antreibbaren Endeffektor (2) zur Übertragung einer zyklischen mechanischen Belastung auf einen Hautbereich (3, 4) einer Hautoberfläche der menschlichen Haut in Verbindung mit einer manuell übertragenen Vorschubkraft in Vorschubrichtung (V) infolge der Betätigung des Handgeräts (1), die ausreicht, um auf ein oder mehrere Hautproteine regulierend einzuwirken, wobei der Endeffektor (2) mehrere, insbesondere eine Vielzahl von reversierend zwischen einer Arbeitsposition und einer Ruhe- bzw. Nichteingriffsposition translatorisch antreibbaren strangförmigen Wirkelemente (5) aufweist, die zyklisch in Kontakt mit der Haut treten, sodass zyklisch in der Ruhe- bzw. Nichteingriffsposition der Kontakt zwischen den Wirkelementen (5) und der Haut unterbrochen ist und die Wirkelemente (5) in eine gegenüber der Hautoberfläche beabstandete Position zurückkehren, und in der Arbeitsposition die Wirkelemente (5) non-invasiv auf die Haut wirken, wobei der Endeffektor (2) und/oder das Handgerät (1) eine zumindest abschnittsweise ebene Anlagefläche (7) zur Anlage mit einer vorbestimmten Orientierung des Endeffektors (2) gegenüber dem Hautbereich (3, 4) aufweist, und die jeweilige Bewegungsachse (Pfeilrichtung 6) der translatorischen Bewegung der Wirkelemente (5) einen spitzen Winkel (a) mit der Anlagefläche (7) einschließt, sodass im Gebrauch über die gesamte, mehrere Wirkelemente (5) einschließende Wirkfläche zyklisch eine homogene Krafteinwirkung erzeugt wird, wobei in jedem Zyklus der Hautbereich (4) in Vorschubrichtung (V) vor der Wirkfläche vorübergehend gegenüber der umgebenden Hautfläche gedehnt, gestaucht und/oder komprimiert wird, indem die Wirkelemente (5) so ausgeführt sind, dass diese in einer ersten Arbeitsphase ihrer translatorischen Bewegung nicht oder nur geringfügig, insbesondere die Epidermis nicht durchdringend, in die Haut eindringen und dabei eine maximale Eindringtiefe (t) erreichen, und in einer zweiten Arbeitsphase der fortgesetzten translatorischen Bewegung die Eindringtiefe (t) nicht oder nur geringfügig erhöht ist und dadurch der Hautbereich (3) relativ zu der Anlagefläche (7) in Vorschubrichtung (V) gedehnt wird.

2. Verfahren zur Hautstraffung, bei dem zyklisch eine mechanische Belastung auf einen Hautbereich (3, 4) einer Hautoberfläche der menschlichen Haut in Verbindung mit einer manuellen Vorschubkraft übertragen wird, die ausreicht, um auf ein oder mehrere Hautproteine regulierend einzuwirken, wobei mehrere strangförmige Wirkelemente (5) reversierend zwischen einer Arbeitsposition und einer Ruhe- bzw. Nichteingriffsposition translatorisch bewegt werden, um mit der Haut in Kontakt zu treten, wobei zyklisch in der Ruhe- bzw. Nichteingriffsposition der Kontakt zwischen den Wirkelementen (5) und der Haut unterbrochen wird, und die Wirkelemente (5) in eine gegenüber der Hautoberfläche beabstandete Position zurückkehren, und in der Arbeitsposition die Wirkelemente (5) non-invasiv auf die Haut wirken, wobei die Wirkelemente (5) in einem spitzen Winkel (a) zu der Hautoberfläche bewegt werden und über die gesamte, mehrere Wirkelemente (5) einschließende Wirkfläche zyklisch eine homogene Krafteinwirkung erzeugt wird, wobei in jedem Zyklus der Hautbereich (4) in Vorschubrichtung (V) vor der Wirkfläche vorübergehend gegenüber der umgebenden Hautfläche gestaucht oder komprimiert ist, indem die Wirkelemente (5) in einer ersten Arbeitsphase ihrer translatorischen Bewegung nicht oder nur geringfügig, die Epidermis nicht durchdringend, bis zu einer maximalen Eindringtiefe (t) in die Haut eindringen, und in einer zweiten Arbeitsphase der insbesondere unterbrechungsfrei fortgesetzten translatorischen Bewegung die Eindringtiefe (t) nicht oder nur geringfügig erhöht wird und dadurch der Hautbereich (3) relativ zu der Anlagefläche (7) in Vorschubrichtung (V) gedehnt wird.

3. Handgerät (1) oder Verfahren nach Anspruch 1 oder 2, **dadurch gekennzeichnet, dass** die Hautbereiche (3, 4) mit unterschiedlichen Vorschubrichtungen (V), insbesondere quer und parallel zu der Orientierung von Hautfalten behandelt werden.

4. Handgerät (1) oder Verfahren nach zumindest einem der vorhergehenden Ansprüche, **dadurch gekennzeichnet, dass** die Frequenz der translatorischen Bewegung der Wirkelemente (5) zwischen der Arbeitsposition und der Ruhe- bzw. Nichteingriffsposition zwischen 10 Hz und 500 Hz, insbesondere zwischen 50 Hz und 100 Hz, einstellbar ist oder eingestellt wird.

5. Handgerät (1) oder Verfahren nach zumindest einem der vorhergehenden Ansprüche, **dadurch gekennzeichnet, dass** die maximale Eindringtiefe (t) der Wirkelemente (5) einstellbar ist oder eingestellt wird.

6. Handgerät (1) oder Verfahren nach zumindest einem der vorhergehenden Ansprüche, **dadurch gekennzeichnet, dass** die Amplitude der translatorischen Bewegung der Wirkelemente (5) und/oder die Eindringtiefe (t) zwischen der Arbeitsposition und der Ruhe- bzw. Nichteingriffsposition weniger als 2 Millimeter, vorzugsweise zwischen 1/100 Millimeter und 1 Millimeter, beträgt.

7. Handgerät (1) nach zumindest einem der vorhergehenden Ansprüche, **dadurch gekennzeichnet, dass** die Wirkelemente (5) nadel- oder spindelförmig ausgeführt sind.

8. Handgerät (1) nach zumindest einem der vorhergehenden Ansprüche, **dadurch gekennzeichnet, dass** die Anlagefläche (7) zur Glättung der Haut infolge der Vorschubkraft quer zu der Vorschubrichtung (V) ausgeführt ist.

9. Handgerät (1) nach zumindest einem der vorhergehenden Ansprüche, **dadurch gekennzeichnet, dass** das Handgerät (1) mit einer Einrichtung zur Begrenzung der manuell übertragenen Kraft in Vorschubrichtung (V) ausgestattet ist.

10. Handgerät (1) nach zumindest einem der vorhergehenden Ansprüche, **dadurch gekennzeichnet, dass** die Anlagefläche (7) mit einem Wirkstoffreservoir verbunden ist.
